# EUROPÄISCHE PATENTANMELDUNG

(11) **EP 3 120 841 A1**
(43) Veröffentlichungstag der Anmeldung: **25.01.2017**
(21) Anmeldenummer: 15178129.1
(22) Anmeldetag: 23.07.2015
(51) Int. Cl.: A61K 31/045, A61K 31/4166, A61K 31/472, A61K 31/573

(54) **PHARMAZEUTISCHES PRÄPARAT**

(71) Anmelder: Sivanzire, Christophe, 14089 Berlin (DE)
(72) Erfinder: Sivanzire, Christophe, 14089 Berlin (DE)
(74) Vertreter: Limbeck, Achim

(57) **Zusammenfassung**

Die vorliegende Erfindung betrifft ein pharmazeutisches Präparat zur Behandlung von Analfissuren, insbesondere zur analen Verabreichung in Zäpfchenform oder als Creme, dadurch gekennzeichnet, dass es zumindest die folgenden Bestandteile umfasst:
a. Papaverin oder ein Papaverinderivat
b. Alpha-Bisabolol
c. Allantoin
d. Prednisolon.

## Beschreibung

### Technisches Gebiet

Die vorliegende Erfindung betrifft ein pharmazeutisches Präparat (Arzneimittel) zur Behandlung von Analfissuren, insbesondere zur analen Verabreichung in Zäpfchenform oder als Creme.

### Stand der Technik

Als Analfissur wird ein sehr schmerzhafter Einriss des Afters bzw. der perianalen Haut bezeichnet und sie betrifft stereotyp die hintere Kommissur des Analkanals, dorsal bei 6 Uhr Steinschnittlage, und verläuft radiär (strahlförmig). Die Analfissur gehört zu den häufigen Enddarmerkrankungen, wobei zwischen der akuten und der chronischen Analfissur unter schieden wird. Betroffen sind vornehmlich jüngere bis mittelalte Erwachsene. Als Symptom steht ein stechender Schmerz während des Einreißens im Vordergrund, wobei dem Akutereignis typischerweise ein Brennen und krampfartiger Nachschmerz sowie gelegentlich Blutungen und/oder ein Juckreiz folgen. Besonders intensive Schmerzen entstehen dabei durch harten Stuhlgang. Bei der Anamnese wird daher nahezu immer eine massive intraanale Druckerhöhung festgestellt.

Besteht eine chronisch erhöhte Muskelspannung des Schließmuskels (Sphinktertonus), kann die verletzte Schleimhaut im Analbereich schlechter abheilen. Starke Schmerzen beim Stuhlgang sorgen wiederum dafür, dass der Schließmuskel sich noch mehr verspannt, was einen Schmerzkreislauf bewirkt, der die Wundheilung verzögert und so in eine chronische Analfissur münden kann, bei der die Symptome länger als zwei Monate vorliegen.

Das Prinzip der konservativen Behandlung auch von chronischen Analfissuren besteht daher vornehmlich in der Entspannung des Schließmuskels, nämlich in der Senkung des intraanalen Druckes durch Aufdehnungsverfahren zur Entlastung des Afterschließmuskels. Die Analdehnung wurde schon jedoch von der modernen Proktologie, auch in Deutschland zur Therapie der Analfissur verboten, weil sie zu einer Analinkontinenz führt. Zur konservativen Therapie der Analfissur existieren auf dem Markt grundsätzlich drei Medikamente, welche als Zäpfchen und/oder Salben auf der Basis von Stickstoffoxid (Nitrogycerin)-Muskelrelaxant und Calciumkanalblocker (Diltiatem) sowie Botulinumtoxin (Botox) verabreicht werden.

Die Giftwirkung der Eiweißstoffe des letztgenannten Botiliums beruht auf der Hemmung der Erregungsübertragung von Nervenzellen, was neben Störungen des vegetativen Nervensystems insbesondere eine Muskelschwäche bis hin zum Stillstand der Lungenfunktion zur Folge haben kann. Botulinumtoxin ist eines der stärksten bekannten Gifte. Diese findet seine Anwendung gegenwärtig seit 1980 in der Medizin vor allem in der Schönheitsmedizin.

Als operative Therapie wird die sog. laterale Sphinterotomie durchgeführt, welche allerdings in den wenigsten Fällen erfolgreich ist und in vielen Fällen sogar zur Entstehung einer irreversiblen Analinkontinenz führt. Bekanntermaßen besitzen die aufgeführten Medikamente darüber hinaus viele Nebenwirkungen, u.a. starke Kopfschmerzen, akuter Abfall des Blutdrucks, allergische Reaktionen usw. Gleichzeitig werden manche von ihnen nicht vollständig resorbiert.

### Darstellung der Erfindung

Der vorliegenden Erfindung liegt die Aufgabe zu Grunde, ein pharmazeutisches Präparat zu schaffen, welches die vorgenannten Nachteile ausräumt und welches geeignet ist, eine Analfissur durch Entspannung des internen Sphinctermuskels, der für die Entstehung der Analfissur als Nekrose des Anoderms durch die Ischämie verantwortlich ist, auszuheilen, ohne dabei auf Substanzen zurückgreifen zu müssen, welche beim Patienten zu Nebenwirkungen führen.

Erfindungsgemäß wird die voranstehende Aufgabe gemäß dem Oberbegriff des Anspruchs 1 in Verbindung mit den kennzeichnenden Merkmalen gelöst. Vorteilhafte Ausgestaltungen und Weiterbildungen des erfindungsgemäßen pharmazeutischen Präparats sind in den abhängigen Unteransprüchen angegeben.

Erfindungsgemäß ist ein pharmazeutisches Präparat der eingangs genannten Art dadurch gekennzeichnet, dass es zumindest die im Folgenden aufgelisteten und näher beschriebenen Bestandteile umfasst.

a. Zunächst bezieht sich die vorliegende Erfindung auf die Verwendung von Papaverin oder eines Papaverinderivats, vorzugsweise Moxaverin zur Herstellung des Arzneimittels. Das bevorzugt zum Einsatz kommende Papaverinderivat Moxaverin ist eine Weiterentwicklung des Wirkstoffes Papaverin und wirkt als muskulotropes Spasmolytikum, welches jedoch eine längere und höhere Wirksamkeit als die Muttersubstanz Papaverin aufweist, weshalb es gegenüber Papaverin vorliegend auch bevorzugt zum Einsatz kommt. Die muskelrelaxierende Wirkung beruht auf der Hemmung der Phosphodiesterase und dem dadurch bedingten Anstieg des zyklischen AMP Spiegels in den glatten Muskelzellen. Darüber hinaus bewirkt der Wirkstoff Moxaverin eine Erhöhung der Erythrozytenflexibilität und führt zur Hemmung der Thrombozytenaggregation. Die Behandlung von Patienten mit Moxaverin ist nach klinischen Studien mit einer hohen klinischen Wirksamkeit und gleichzeitiger hoher Arzneimittelsicherheit verbunden, wobei der Wirkstoff zusätzlich eine hohe Verträglichkeit aufweist. Der Gehalt an Papaverin oder eines Papaverinderivats, insbesondere an Moxaverin, welcher vorzugsweise in der Form des Hydrochlorids vorliegt, beträgt vorliegend vorzugsweise mindestens 0,025 mg/ml.

b. Als weiteren wesentlichen Bestandteil beinhaltet das pharmazeutische Präparat Alpha Bisabolol. Alpha Bisabolol ist ein kamillepflanzliches Extrakt. Kamillenblüten gelten als unverzichtbar in der Behandlung von Haut- und Schleimhautentzündungen und zur Wundbehandlung bei Schnitt-, Schürf-oder Platzwunden sowie bei Verbrennungen und trockenen Wunden mit Krustenbildung. Ihr Hauptwirkstoff, das Alpha Bisabolol, hemmt die Cycloxygenase und die Lipoxygenase, wodurch Botenstoffe gehemmt werden, welche die Entzündungsreaktion unterstützen. Kamillenblüten-Extrakt wirkt darüber hinaus gegen Bakterien wie Staphylokokken, weshalb es auch bei infizierten Wunden unter ärztlicher Aufsicht verwendet werden kann. Weiterhin ist eine Wirkung gegen Viren (Herpes), Pilze und als Immunstimulans bekannt. Der Gehalt an Alpha Bisabolol beträgt vorliegend vorzugsweise mindestens 0,5 mg/ml.

c. Als weiteren Bestandteil weist das vorliegende Präparat Allantoin auf. Allantoin betrifft ein Stoffwechselprodukt, das u. a. in Wallwurz, Ahorn, Schwarzwurzeln, Rüben, Rosskastanien und in Weizenkeimen vorkommt. Es kommt in pflanzlichen und tierischen Organismen als Endprodukt des Purinstoffwechsels vor und wird aus Harnsäure gebildet. Allantoin gehört zur Gruppe der Imidazolidine und wird beim oxydativen Abbau von Harnsäuren von Enzym Uricase gebildet. Allantoin fördert Zellproliferation, die Epithelbildung und entfernt nekrotisches Gewebe. Es hat anabole, antioxidative, feuchtigkeitsspendende, glättende, keratolytische, penetrationsfördernde und antimutagene Eigenschaften. Allantoin wird äußerlich zur Haut- und Narbenpflege, zur Wundbehandlung, gegen Hauterkrankungen und als Kosmetikum eingesetzt. In Arzneimitteln ist es häufig enthalten, um die Penetration von Wirkstoffen durch die Haut zu fördern. Allantoin gilt als wundheilungsfördernd und zellgenerierend und hat eine keratoplastische (das bedeutet Hornhaut erweichende) Wirkung. Der Bestandteil Allantoin ist allgemein äußerst verträglich und eignet sich hervorragend für entzündliche Prozesse. Allantoin wird vorliegend aufgrund seiner intensiven Wirksamkeit in einer geringen Dosierung von maximal 0,5% eingesetzt. Der Bestandteil Allantoin am Präparat beträgt vorzugsweise 0,5 mg/ml.

d. Prednisolon, welches auch als 1,2-Dehydrocortisol; 11alpha, 17,21-Trihydroxy-1,4-pregnadien-3,20-dion; Deltacortisol; Prednisolonum bezeichnet wird, bildet einen weiteren Bestandteil des Präparats, dessen Bestandteil am Präparat vorzugsweise bei 0,008 mg/ml liegt. Das Prednisolon verhindert eine mögliche allergische Reaktion, die meistens im Analbereich als Reaktion auf atopische Behandlungsmedikamente oder verschiedene Therapieverfahren entstehen.

In weiteren bevorzugten Ausführungsformen der Erfindung werden dem Präparat zusätzlich entweder Oleum Cacao und/oder Laurinsäure in geringer Dosis zugesetzt.

Das erfindungsgemäße Präparat wurde in einer Pilotstudie getestet, welche in einigen Krankenhäusern in Deutschland und in einer Privatpraxis in Polen durchgeführt wurde. Die Ergebnisse dieser Studie seien nachstehend kurz dargestellt.

Bei 176 Patienten wurde in zwei Jahren mit vollständig diagnostzierter Analfissur Proctocalm verabreicht. Dabei litten 32 Patienten an chronischer Obstipation, 37 an einer mechanischen Verletzung des Anus, 27 an akuter Diarrhoe, 3 an M. Crohn, 12 an einem perianalem Infekt sowie 8 Patienten an einer Analfistel. Darüber hinaus waren 42 Patienten Raucher, 52 litten unter chronischem Stress, 97 hatten eine labile Psyche, 115 berichteten über ballastarme Diät, 37 Patienten über eine überwiegend sitzende Tätigkeit und 22 Patienten über eine im Vorfeld der Studie durchgeführte operative Behandlung im Analbereich.

Bei 101 Patienten lagen die Fissuren bei 6 Uhr in SSL, 15 bei 12 Uhr, 23 bei 6 und 12 Uhr in SSL, 7 in verschiedenen Lagerungen im Analbereich.

Die Therapiedauer bis zur völligen Ausheilung der Analfissur lag bei 63 Patienten bei 2 Wochen, bei 40 Patienten bei 3 Wochen, bei 17 bei 4 Wochen, bei 12 Patienten bei 5 Wochen, bei 6 Patienten bei 6 Wochen, bei 2 bei 7 Wochen, bei 4 Patienten bei 8 Wochen und bei 2 Patienten eine Therapieresistenz, so dass eine subkutane Fissurotomie erfolgen musste.

Die Linderung der Schmerzsymptomatik erfolgte bei 3 Patienten bereits am ersten Tag nach der Verabreichung des Proctocalms, bei 4 Patienten am 2 Tag, bei 71 Patienten am 3 Tag, bei 63 Patienten am 4 Tag, bei 2 Patienten am 2 Tag, bei 1 Patient am 6 Tag, und 1 am 7 Tag.

Eine allergische Reaktion trat bei 1 Patient ein.

Gemäß dieser Pilotstudie zeigt Proctocalm bessere Heilungsergebnisse als herkömmliche Maßnahmen.

Das erfindungsgemäße Präparat beschränkt sich in seiner Ausführung nicht auf die vorstehend angegebenen bevorzugten Ausführungsformen. Vielmehr sind eine Vielzahl von Ausgestaltungsvariationen denkbar, welche von der dargestellten Lösung auch bei grundsätzlich anders gearteter Ausführung Gebrauch machen.

## Patentansprüche

1. Pharmazeutisches Präparat zur Behandlung von Analfissuren, insbesondere zur analen Verabreichung in Zäpfchenform oder als Creme,
**dadurch gekennzeichnet, dass**
es zumindest die folgenden Bestandteile umfasst:
a. Papaverin oder ein Papaverinderivat
b. Alpha-Bisabolol
c. Allantoin
d. Prednisolon.

2. Präparat nach Anspruch 1,
dadurch gekenntzeichnet, dass
das Papaverinderivat Moxaverin ist.

3. Präparat nach einem der vorangegangenen Ansprüche,
dadurch gekenzeichret, dass
Papaverin oder das Papaverinderivat in der Form des Hydrochlorids vorliegt.

4. Präparat nach einem der vorangegangenen Ansprüche,
**dadurch gekennzeichnet, dass**
es Oleum Cacao enthält.

5. Präparat nach einem der vorangegangenen Ansprüche,
dadurch gekenntzeichnet, dass
es zusätzlich Laurinsäure enthält.

6. Präparat nach einem der vorangegangenen Ansprüche,
**dadurch gekennzeichnet, dass**
der Gehalt an Moxaverin in der Form des Hydrochlorids mindestens 2,5 mg/ml beträgt.

7. Präparat nach einem der vorangegangenen Ansprüche,
**dadurch gekennzeichnet, dass**
der Bestandteil an Prednisolon bei 0,008 mg/ml liegt.

8. Präparat nach einem der vorangegangenen Ansprüche,
**dadurch gekennzeichnet, dass**
Allantoin in einer Dosierung von maximal 0,5% eingesetzt ist.

9. Präparat nach einem der vorangegangenen Ansprüche,
**dadurch gekennzeichnet, dass**
der Bestandteil an Allantoin bei 0,5 mg/ml liegt.
